# EUROPEAN PATENT SPECIFICATION

(11) **EP 4 135 684 B1**
(45) Date of publication and mention of the grant of the patent: **18.06.2025**
(21) Application number: 21788400.6
(22) Date of filing: 15.04.2021
(51) Int. Cl.: A61K 9/107, A61K 31/232, A61K 47/24, A61K 47/14, A61P 9/10

(54) **STABLE DIGLYCERIDE EMULSIONS AND METHODS FOR TREATING ORGAN INJURY**
STABILE DIGLYCERIDEMULSIONEN UND VERFAHREN ZUR BEHANDLUNG VON ORGANVERLETZUNGEN
ÉMULSIONS DE DIGLYCÉRIDE STABLES ET MÉTHODES DE TRAITEMENT D'UNE LÉSION D'UN ORGANE

(30) Priority: 15.04.2020 US 202063010364 P
(43) Date of publication of application: 22.02.2023
(73) Proprietor: The Trustees of Columbia University in the City of New York, New York, NY 10027 (US)
(72) Inventor: DECKELBAUM, Richard J., Hudson, New York 10706 (US); CHANG, Chuchun Liz, New York, New York 10024 (US); ZIRPOLI, Hylde, Long Island City, New York 11101 (US)
(74) Representative: Marks & Clerk LLP
(86) International application number: PCT/US2021/027411
(87) International publication number: WO 2021/211796

(56) References cited:
- US-A1- 2012 251 685
- US-A1- 2012 251 685
- US-A1- 2015 011 515
- US-A1- 2015 011 515
- US-A1- 2016 206 622
- US-B2- 8 410 181
- ANONYMOUS: "Diglyceride", 26 October 2019 (2019-10-26), pages 1 - 4, XP055865786, Retrieved from the Internet <URL:https://en.wikipedia.org/w/index.php?title=Diglyceride&oldid=923070725> [retrieved on 20210614]
- ANONYMOUS: "Dispersity", 10 April 2019 (2019-04-10), pages 1 - 4, XP055865793, Retrieved from the Internet <URL:https://en.wikipedia.org/w/index.php?title=Dispersity&oldid=891756923> [retrieved on 20210614]

## Description

### BACKGROUND

Stroke is the leading cause of long-term disability in the United States and the 5th leading cause of death. To date, tissue plasminogen activator (t-PA) remains the only FDA-approved drug for acute ischemic stroke treatment. However, its use is limited by a narrow 3 to 4.5 hour time window.

Omega-3 (n-3) fatty acids (FAs) are candidates for acute neuroprotection after stroke. Evidence suggests that n-3 FAs act as bioactive unsaturated lipids with pleiotropic effects and show neuroprotective properties in animal models of stroke. A number of biological mechanisms may be affected by n-3 FAs, including (i) decrease in generation of mitochondrial reactive oxygen species (ROS); (ii) preservation of mitochondrial Ca²⁺ uptake and homeostasis; (iii) modulation of receptor-mediated signal transduction and inhibition of apoptotic pathways; (iv) increase in potent n-3 FA-derived resolvins and protectins, and (v) decrease in inflammatory responses. Working separately or synergistically, these mechanisms may contribute to n-3 FA neuroprotection in ischemic injury, decreasing cell death while accelerating repair processes.

However, to be effective for neuroprotection, adequate levels of n-3 FAs must be quickly delivered to cells at risk of cell death or injury. This disclosure in certain aspects provides compositions and methods for acute delivery of n-3 FAs for treatment of organ damage, including for neuroprotection. The invention finds use as an emergency medicine for the treatment of stroke, myocardial infarction, traumatic brain injury, and ischemic organ injuries among others.

### SUMMARY OF INVENTION

The present invention provides compositions and methods involving stable n-3 diglyceride (DG) oil-in-water emulsions for acute therapy to treat and/or prevent organ injury. The compositions provide protection from cellular death, and find use in patients in need of neuroprotection or organ protection, including for ischemic stroke, myocardial infarction and traumatic brain injury, among others. The compositions have a large time window by which administration is effective after onset of injury (e.g., after onset of stroke). The compositions may be administered in conjunction with other therapies, and the compositions may be administered during a recovery phase to further improve outcomes.

In various aspects and embodiments, the compositions are stable emulsions that can be stored in stable form for use in the emergency setting. For example, in various embodiments, the compositions will be delivered on-site by emergency medicine professionals. The emulsions described herein are substantially stable for at least six months, or at least one year, or at least 24 months. The compositions are suitable for parenteral delivery, such as intravenous (i.v.) or intra-arterial delivery, as well as via intragastric or intraduodenal tubes, and the physical properties of the emulsions facilitate rapid delivery of the n-3 FAs for uptake by damaged tissue, including brain tissue.

In accordance with the invention, the esterified FAs of the DG are predominately n-3 FAs. The DG comprises at least about 90% n-3 FAs, or about 100% n-3 FAs, comprising docosahexaenoic acid (DHA) and eicosapentaenoic acid (EPA). In some embodiments, the n-3 fatty acids are DHA and EPA.

The stable emulsions have a mean particle size of from 110 to 180 nm or less and a zeta potential (ZP) of about -30mV, or more negative than -30 mV. In some embodiments, the mean particle size of the emulsions is about 180 nm or less, or about 160 nm or less, or about 140 nm or less. In various embodiments, the polydispersity index (PDI) is 0.3 or less. In various embodiments, the zeta potential of the emulsions is at least as negative as about -45 mV, or at least as negative as about -50 mV, or at least as negative as about -55 mV, or at least as negative as about -60 mV.

The stable emulsions are suitable for parenteral or enteral administration for example, to rapidly deliver n-3 FAs to injured cells and tissues, including in some embodiments the brain. The lipid component of the emulsions will generally be from about 10% to about 50% by weight of the composition. In some embodiments, at least about 20% by weight of the composition is DG oil, and in some embodiments the composition is from about 23% to about 30% by weight DG oil. The composition will also include emulsifiers and optionally co-emulsifiers as described herein.

The compositions will comprise one or more emulsifiers to obtain the desired physical characteristics. In various embodiments, emulsifiers can include one or more of phospholipid emulsifiers, phosphoglyceride emulsifiers, and medium and/or long chain fatty acid emulsifiers. In various embodiments, the composition comprises less than about 1% by weight of emulsifiers.

An exemplary composition according to this disclosure, is a composition suitable for intravenous or intra-arterial injection, where the composition comprises a stable diglyceride (DG) oil-in-water emulsion. The emulsion comprises 10% by weight of a DG oil, the esterified fatty acids of the DG oil being at least about 90% n-3 fatty acids and comprising DHA and EPA, and wherein the emulsion has a mean particle size of from 110 to 180 nm with a polydispersity index of 0.3 or less and a zeta potential of about -40 mV or more negative than -40 mV.

In various embodiments, the composition is approximately isotonic with human blood, and optionally comprises one or more polyols, such as glycerol, sorbitol, xylitol, and/or glucose. In some embodiments, the composition comprises one or more anti-oxidants, such as one or more of α-tocopherol, β-tocopherol, γ-tocopherol, and an ascorbyl ester. In exemplary embodiments, the anti-oxidants comprise α-tocopherol and/or ascorbyl ester, which is optionally ascorbyl palmitate. In some embodiments, the composition comprises a metal chelating agent, which is optionally ethylenediamine tetraacetic acid (EDTA) or ethyleneglycol-bis-(β-aminoethylether)-*N*,*N*,*N*',*N*'-tetraacetic acid (EGTA).

The emulsions can also be co-formulated with other lipophilic active agents, to enhance delivery of these otherwise difficult to deliver therapies, and which can provide synergistic results with other mechanisms of action. For example, in some embodiments the emulsions are co-formulated with glibenclamide or a statin. The DG compositions can be administered with one or more additional neuroprotectants. In still other embodiments, these additional agents are administered separately from the emulsions as co-therapy.

In other aspects, the invention provides a method for treating a patient in need of protection from cellular death, including acute and chronic injuries to various organs or tissues, such as the brain, spinal cord, and newly transplanted organ, among others. In some embodiments, the patient is in need of treatment for an ischemic organ injury, or in need of protection from damage from an ischemic organ injury.

Thus, in some embodiments the patient is experiencing stroke. The compositions described herein can be administered after stroke onset to provide neuroprotection, that is, inhibit cellular processes leading to cell death. The physical and chemical properties of the emulsions allow them to be effective, even when delivered later than desired after stroke onset. For example, in various embodiments, the patient is administered the composition within about twelve hours of stroke onset.

The compositions are compatible for treating both ischemic and hemorrhagic stroke, and thus can be administered by emergency personnel, that is prior to brain imaging to detect or visualize a clot or potential hemorrhage. In the absence of hemorrhage, the patient may receive thrombolytic therapy to dissolve the clot (e.g., t-PA). While t-PA conventionally is administered to a stroke victim within about the first 4.5 hours after a stroke occurs, in accordance with the present disclosures, the patient receives such thrombolytic therapy after about 4.5 hours from stroke onset. By administering the emulsion compositions as soon as possible in the emergency setting, more time can be obtained to determine whether thrombolytic therapy is appropriate. In still other embodiments, a thrombectomy is performed. The compositions described herein can expand the therapeutic window where thrombectomy is successful.

In some embodiments, in the context of ischemic stroke, the subject may receive a dose of the DG emulsions as soon as possible after the onset of stroke, and generally within about 24 hours, or within about 12 hours, or within about 10 hours, or within about 8 hours, or within about 6 hours. The patient may receive subsequent doses of the DG emulsions and/or oral supplementation with n-3 DGs and/or n-3 triglycerides (TGs) over the following days, weeks, or months to aid recovery.

In other embodiments, the patient is suffering from or at risk of traumatic brain injury (TBI). For example, the patient may be administered the composition within 1 to 24 hours after brain injury, to reduce long term tissue damage from TBI. In some embodiments, after the initial administration, the patient is administered the composition with a frequency of from about once every four hours to about once per week to aid recovery. The patient may optionally receive oral supplementation with n-3 DGs and/or n-3 TGs over the following days, weeks, or months to aid recovery.

In still other embodiments, the patient is suffering from post-traumatic stress disorder (PTSD). For example, the patient may be administered the composition with a frequency of at least about once per week for a period of time to facilitate recovery. The patient may optionally receive oral supplementation with n-3 DGs and/or n-3 TGs to support recovery.

The invention provides for protecting other organs or tissues, including ischemic and traumatic tissue injuries, including spinal cord injury (SCI). In such embodiments, the patient may be administered the composition shortly after injury in the emergency setting. In some embodiments, the patient is administered the composition with a frequency of at least once per day to once per week after the initial administration. The patient may optionally receive oral supplementation with n-3 DGs and/or n-3 TGs over the following weeks or months to aid recovery. For example, the patient may receive oral supplementation at least once daily.

Further, in some embodiments, the patient is the recipient of an organ transplant, such as liver, kidney, heart, intestinal or lung transplant. In some embodiments, the patient is administered the composition at least once during the perioperative period. After the perioperative period, the patient may be administered the composition at frequencies ranging from about once every four hours to once per week to aid recovery. In still other embodiments, the patient is treated for acute organ failure, including acute renal, liver, or heart failure. The patient may optionally receive oral supplementation with n-3 DGs and/or n-3 TGs for one or more weeks to one or more months following transplant to support recovery.

In some embodiments, the patient is suffering from a neurodegenerative disease. For example, the patient having a neurodegenerative disease is administered the composition at least once per week to slow disease progression, and/or is administered the composition after disease relapse.

Other aspects and embodiments of the invention will be apparent from the following examples.

### DESCRIPTION OF THE FIGURES

**FIG. 1** shows the physical characteristics of DG emulsions prepared in accordance with this disclosure, and containing both DHA and EPA, and triglyceride (TG) emulsions. FIG. 1, left, shows mean particle size (diameter). FIG. 1 (right) shows zeta potential (ZP).
**FIG. 2A** and **FIG. 2B** show total free fatty acids (FFA) released by hydrolysis of 200 µg of DG or TG emulsions measured by colorimetric assay and expressed in nanomoles. (FIG. 2A) Tri-DHA 10% PL 1.2% vs DG 10% PL 1.2%. (FIG. 2B) Tri-DHA 20% PL 1.2% vs DG 20% PL 1.2%.
**FIG. 3** shows TLC analyses for n-3 DG oil to test purity and integrity and to identify 1,2-1,3 DG species.
**FIG. 4A** and **FIG. 4B** show (FIG. 4A) infarct volume in neonatal mice (10-day old) subjected to HI injury and treated with saline as vehicle (black bar) or emulsions: n-3 TG (gray bar), n-3 DG (dark gray bar) or n-6 DG (light gray bar). N=15-17. Values are mean ±SD; (FIG. 4B) TTC stained brain sections. *p< 0.05; **<0.01 (ANOVA) compared to saline. Doses = 0.375 mg/g body weight.
**FIG. 5A** and **FIG.** show (FIG. 5A) infarct volume in adult mice subjected to middle cerebral artery occlusion (MCAo) and treated with saline or n-3 DG emulsion. Values are mean ±SEM. N=5 (FIG. 5B) TTC stained brain sections. *p<0.05 (student's t-test) compared to saline group. Dose per mouse = 100 mg.
**FIG. 6A** and **FIG. 6B** show (FIG. 6A) therapeutic time window in rats subjected to MCAo and treated with saline or n-3 TG emulsion at 6 and 8 hours after ischemia. Values are mean ±SEM. N=6; (FIG. 6B) TTC stained brain sections. *p<0.05 (ANOVA) compared to saline group. Dose per rat = 150 mg.
**FIG. 7** shows average infarct volumes in mice treated immediately after ischemic injury with either Saline, DHA, EPA, DHA-EPA, or ARA (all DG emulsions at doses of 0.375g DG /Kg).

### DETAILED DESCRIPTION

The present invention provides compositions and methods involving stable n-3 DG oil-in-water emulsions for acute therapy to treat and/or prevent tissue or organ injury. The compositions provide protection from cellular death, and find use in patients in need of neuroprotection or organ protection. For example, the compositions find use in treating ischemia reperfusion injuries, such as ischemic stroke and myocardial infarction. The compositions further find use for treatment of traumatic injuries, such as traumatic brain injury or spinal cord injury, among others. The compositions have a large time window by which they are effective after onset of traumatic or ischemic injury (e.g., after onset of stroke), and may be administered in conjunction with other therapies.

In various aspects and embodiments, the compositions are stable emulsions that can be stored in stable form for use in the emergency setting. For example, in various embodiments, the compositions will be delivered on-site by emergency medicine professionals. The emulsions described herein are substantially stable for at least six months, or at least one year, or at least 18 months, or at least two years, in various embodiments. The compositions are suitable for parenteral delivery routes, such as intravenous or intra-arterial delivery. Further, in some embodiments the physical properties of the emulsions, such as mean particle diameters of 200 nm or less, facilitate delivery of the n-3 fatty acids to, and/or uptake by, brain tissue. Without being bound by theory, it is believed that this small particle size will improve rapid delivery to the brain, which is critical for neuroprotection in conditions such as stroke.

Emulsions are inherently unstable and, thus, do not form spontaneously. Energy input through shaking, stirring, homogenizing, for example, is needed to form an emulsion. Over time, emulsions tend to revert to the stable state of the phases comprising the emulsion. However, nanoemulsions can be kinetically stable.

If the size and dispersion of droplets of an emulsion does not substantially or significantly change over a desired time frame (such as at least about six months), the emulsion is said to be stable. That is, emulsion stability refers to the ability of an emulsion to resist changes in its properties over time. Instability in emulsions can be observed as, for example, flocculation, creaming/sedimentation, and coalescence. Flocculation occurs when there is an attractive force between the droplets, so they form flocs. Coalescence occurs when droplets combine to form a larger droplet, so that the average droplet size increases over time. Emulsions can also undergo creaming, where the droplets rise to the top of the emulsion under the influence of buoyancy, for example. Sedimentation is the opposite phenomenon of creaming and normally observed in water-in-oil emulsions. Sedimentation happens when the dispersed phase is denser than the continuous phase and the gravitational forces pull the denser globules towards the bottom of the emulsion. Similar to creaming, sedimentation follows Stokes' law.

An emulsifier is a substance that stabilizes an emulsion by increasing its kinetic stability. Emulsifiers include surface active agents, or surfactants. Surfactants can increase the kinetic stability of an emulsion so that the size of the droplets does not change significantly with time. The stability of an emulsion can be evaluated in terms of zeta potential, which indicates the repulsion between droplets or particles. Emulsifiers are compounds that typically have a polar or hydrophilic (i.e. water-soluble) part and a non-polar (i.e. hydrophobic or lipophilic) part. Detergents are a type of emulsifier, and will interact physically with both oil and water, thus stabilizing the interface between the oil and water droplets in suspension.

The present invention delivers n-3 FAs to cells as stable DG emulsions. The term "n-3 FAs" means a polyunsaturated FA where one of the carbon-carbon double bonds is between the third and fourth carbon atoms from the distal end of the hydrocarbon chain. Examples of n-3 FAs include α-linolenic acid (18:3n-3; α-ALA; Δ^{3,6,9}), eicosapentaenoic acid (20:5n-3; EPA; Δ^{5,8,11,14,17}), docosahexaenoic acid (22:6n-3; DHA; Δ^{4,7,10,13,16,19}) and docosapentaenoic acid (22:5n-3; DPA; Δ^{7,10,13,16,19}). n-3 FAs having at least 20 carbon atoms are referred to as "long chain n-3 FAs". Sources of n-3 FAs may be from any suitable source such as from fish oils, algae oils and other oils, or may be synthesized.

A number of biological mechanisms are affected by n-3 FAs that can be beneficial in acute injury, including (i) decrease in generation of mitochondrial ROS; (ii) preservation of mitochondrial Ca²⁺ uptake and homeostasis; (iii) modulation of receptor-mediated signal transduction and inhibition of apoptotic pathways; (iv) increase in potent n-3 FA-derived resolvins and protectins, and (v) decrease in inflammatory responses. Working separately or synergistically, these mechanisms can contribute to n-3 FA neuroprotection in ischemic injury, decreasing cell death while accelerating repair processes.

DGs are composed of two FAs esterified to the trihydric alcohol glycerol. An exemplary method for synthesis of DG molecules is through lipase-catalyzed glycerolysis (i.e., transesterification) with n-3 long chain FAs. In various embodiments, the compositions described herein are substantially DG, that is, such compositions do not contain large amounts of triglycerides. In some embodiments, the emulsion compositions are at least about 75%, or at least about 85%, or at least about 90%, or at least about 95% DG emulsions, with respect to the total amount of DGs and TGs present in the composition.

In accordance with the invention, the FAs of the DGs may be predominately n-3 FAs. In various embodiments, the DG comprises at least about 90% n-3 FAs, or at least 95% n-3 FAs, or about 100% n-3 FAs. In some embodiments, the n-3 FAs are long chain n-3 FAs, including one or more of DHA, EPA, and DPA.

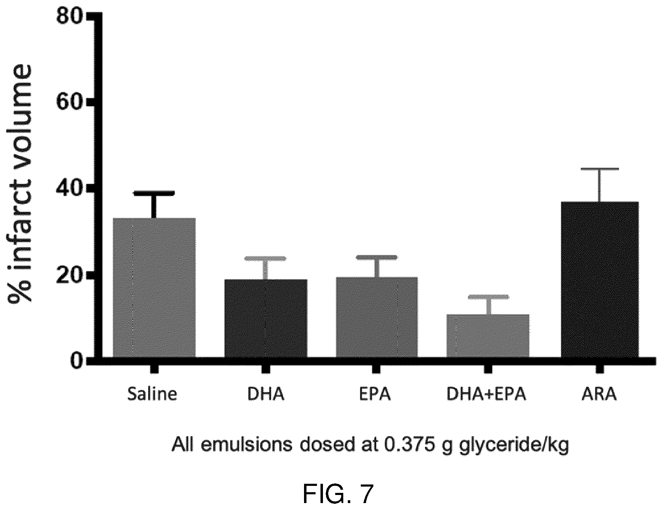

In various embodiments, the n-3 FAs comprise DHA, EPA, and/or DPA. For example, in some embodiments, the n-3 FAs comprise DHA. In some embodiments, the n-3 FAs are at least about 50% DHA, or at least about 60% DHA, or at least about 75% DHA, or at least about 90% DHA. In some embodiments, the n-3 FAs comprise EPA. For example, the n-3 FAs may be at least about 50% EPA, or at least about 60% EPA, or at least about 75% EPA, or at least about 90% EPA. In some embodiments, the n-3 FAs comprise DPA. For example, the n-3 FAs may be at least about 50% DPA, or at least about 60% DPA, or at least about 75% DPA, or at least about 90% DPA. In some embodiments, the n-3 FAs comprise DHA and EPA, which are optionally present at a ratio of from about 2:1 to about 1:2 (e.g., about 1:1). As demonstrated herein, DG emulsions having a small particle size and carrying DHA+EPA show exceptionally high properties in neuroprotection. See FIG. 7.

In various embodiments, the DG molecules comprise 1,3-DGs and 1,2-DGs. In some embodiments, the DGs are predominately 1,3-DGs.

In various embodiments, the emulsions have a mean particle size of 200 nm or less and a zeta potential of about -30 mV or more negative than about -30 mV. In some embodiments, the mean particle size of the emulsions is about 180 nm or less, or about 170 nm or less, or is about 160 nm or less, or is about 150 nm or less, or is about 140 nm or less, or is about 120 nm or less, or about 100 nm or less, or about 90 nm or less, or about 80 nm or less. In some embodiments, the mean particle size is about 140 nm, about 120 nm, or about 110 nm, and with a polydispersity index of less than about 0.3, or less than about 0.25, or less than about 0.2. In some embodiments, the mean particle size is from about 120 nm to about 180 nm, with a polydispersity index of less than about 0.3. In various embodiments, the zeta potential of the emulsions is at least as negative as about -35 mV, or at least as negative as about -40 mV, or at least as negative as about - 50 mV, or at least as negative as about -55 mV. The emulsions in accordance with these embodiments are stable, meaning these parameters are maintained for at least six months, or in some embodiments, at least one year, at least 18 months, or at least two years. In accordance with this disclosure, stability is determined with storage at 4°C.

The stable emulsions are suitable for i.v. administration for example, to rapidly deliver n-3 FAs to injured tissues, including in some embodiments the brain. Thus, in such embodiments the composition is an injectable composition. The lipid component will generally be from about 10% to about 50% by weight of the composition. In some embodiments, the lipid component of the composition will be about 10% to about 30%, or about 15% to about 25%. In some embodiments, the lipid component is from 20% to about 40% by weight of the composition, or from about 20% to about 30%. For example, the lipid component may be at least about 10%, or at least about 15%, or at least about 20% of the composition by weight, or at least about 25% of the composition by weight, or at least about 30% of the composition by weight. In such embodiments, 10% by weight of the composition is DG oil.

Polydispersity index (PDI) is a measure of particle size distribution within a given sample. The numerical value of PDI ranges from 0.0 (for a sample with perfectly uniform particle size distribution) to 1.0 (for a highly polydisperse sample with multiple particle size populations). In lipid-based carriers, such as emulsions, a PDI of 0.3 is desired, indicating a sufficiently homogenous particle size distribution. In some embodiments, the PDI of the emulsions is less than about 0.30, such as about 0.25 or less, 0.20 or less, or about 0.15 or less.

The compositions will comprise one or more emulsifiers to obtain the desired physical characteristics. In various embodiments, emulsifiers can include one or more of phospholipid emulsifiers, phosphoglyceride emulsifiers, and medium and/or long chain fatty acid emulsifiers. In various embodiments, the composition comprises from about 0.5% to about 2.4% by weight of emulsifiers (e.g., phospholipid emulsifiers), such as from about 0.5% to about 2%, and optionally less than about 1.0% by weight of emulsifiers, and optionally from 0.5% to 0.8% of emulsifiers by weight (e.g., phospholipid emulsifiers).

In some embodiments, emulsions comprise one or more phospholipid emulsifiers and/or one or more phosphoglyceride emulsifiers. Phosphoglyceride emulsifiers may be selected from phosphatidylcholine, phosphatidylethanolamine, phosphatidylinositol, phosphatidylserine, and phosphatidic acid. In some embodiments, the composition comprises a phosphatidylcholine emulsifier. In various embodiments, the ratio of phospholipid and/or phosphoglyceride emulsifier to DG (by weight) is 1:8 or less, or is 1:10 or less, or is 1:12 or less, or is 1:15 or less. In some embodiments, the emulsifier comprises at least about 70% phosphatidylcholine, or comprises at least about 80% phosphatidylcholine. For example, the emulsifier (with any co-emulsifier) may contain from about 60% to about 80% phosphatidylcholine.

The composition may further comprise one or more of medium chain or long chain FAs as co-emulsifier. For example, the composition may comprise a long chain FA, optionally selected from a C16 to C24 FA, and which is optionally a C18 FA. In some embodiments, the co-emulsifier comprises a saturated FA, optionally selected from lauric acid, myristic acid, palmitic acid, and stearic acid. In some embodiments, the co-emulsifier comprises an unsaturated FA, optionally selected from oleic acid or linolenic acid. The co-emulsifier may be added as an alkali metal salt, which optionally comprises sodium oleate. In exemplary embodiments, the co-emulsifier is present at about 0.01% to 5% of the total weight of the composition. For example, the co-emulsifier may be present at from about 0.01 to 2% of the total weight of the composition, or from about 0.01% to about 1% of the total weight of the composition, or from about 0.01% to about 0.05% by weight of the composition.

In various embodiments, the composition is approximately isotonic with human blood, and optionally comprises one or more polyols, such as glycerol, sorbitol, xylitol, and/or glucose. For example, the composition may comprise glycerol at from about 2% to about 10% by weight of the composition, or from about 2% to about 7% by weight of the composition.

In some embodiments, the composition comprises one or more anti-oxidants, such as one or more of α-tocopherol, β-tocopherol, γ-tocopherol, and an ascorbyl ester. In exemplary embodiments, the anti-oxidants comprise α-tocopherol and/or ascorbyl ester, which is optionally ascorbyl palmitate.

In some embodiments, the composition comprises a metal chelating agent, which is optionally EDTA or EGTA. For example, emulsions may contain from about 5 mM to about 15 mM EDTA or EGTA. For example, in some embodiments, the emulsions contain about 10 mM EDTA.

In various embodiments, stable emulsions can be prepared according to a process comprising: (1) preparing a mixture of water, glycerol, and EDTA having a temperature of from about 50°C to about 80°C (e.g., about 60°C); (2) add phosphatidylcholine emulsifier (e.g., at least about 75% PC, which may be from egg yolk lecithin), co-emulsifier (e.g., sodium oleate), and DG oil; (3) homogenize at a temperature of from about 50°C to about 80°C (e.g., about 60°C); (4) process through a microfluidizer or for larger volumes, a high pressure homogenizer (i.e., a high shear fluid processor). The pressure applied during this process could range from 300 to 2000 bar, and in some embodiments, from about 500 to about 1000 bar, such as from about 600 to about 1000 bar. For example, the mixture can be processed through the microfluidizer at about 950-bar pressure at about 60°C. The emulsions can be processed for a length of time and under conditions required to meet the target particle size. This process can include co-formulation of other lipophilic agents as described below.

The emulsions can also be co-formulated with other lipophilic active agents, to enhance their delivery and provide synergistic results with other mechanisms of action. For example, in some embodiments the emulsions are co-formulated with glibenclamide. Simard et al., Glibenclamide in cerebral ischemia and stroke. Neurocrit care 2014 20(2):319-333. However, glibenclamide is inefficiently delivered to the brain, and is generally difficult to deliver given its lipophilic nature. Thus, the present disclosure provides DG emulsions to improve delivery of glibenclamide for neuroprotection. Because glibenclamide is a lipophilic active agent, it will readily incorporate into the emulsion, and may be delivered at a lower amount than delivery without emulsion, so that this active is delivered within its therapeutic window.

The emulsion might also be co-formulated with other lipophilic agents such as statins, thereby expanding the benefits of these emulsions to aid recovery and prevent and/or treat chronic disease states, including those where the patient is at risk of ischemic injury, such as atherosclerosis and those at risk for myocardial infarction. Examples of lipophilic statins include atorvastatin, fluvastatin, lovastatin, simvastatin and cerivastatin.

The emulsions in some embodiments further comprise one or more neuroprotectants. In some embodiments, one or more neuroprotectants are administered separately as co-therapy. Exemplary neuroprotectants include glutamate antagonists. Exemplary neuroprotectants include 17β-Estradiol, ginsenosides, progesterone, simvastatin, and memantine. Lipophilic neuroprotectants (e.g., 17β-Estradiol, simvastatin, or progesterone) can be incorporated into the emulsions.

In some embodiments, the emulsions further comprise one or more metabolites of EPA, DHA, and/or DPA, such as one or more resolvins or protectins. Resolvins are polyunsaturated fatty acid (PUFA) metabolites derived from omega-3 fatty acids, including EPA, DHA, and DPA. Resolvins (such as RvD and/or RvE) may promote restoration of normal cellular function following tissue inflammation. Protectins, such as neuroprotectin D1 (NPD1), are also PUFA metabolites that possesses strong anti-inflammatory, anti-apoptotic, and neuroprotective activity. In some embodiments, the emulsions comprise DHA and EPA (as described) with NPD1.

In various embodiments, the pH of the composition is from about 6 to about 10, and optionally from about 6.5 to about 10, and optionally from about 9 to about 10 (e.g., 9.5).

In various embodiments, the composition has a volume of about 500 mL or less, or a volume of about 300 mL or less, or a volume of about 100 mL or less, or a volume of about 50 mL or less, or a volume of about 25 mL or less. In various embodiments, the composition is contained in a pre-filled syringe, optionally having a volume for injection of from about 1 mL to about 50 mL. In some embodiments, the composition is packaged in vials at a volume of from about 25 mL to about 100 mL.

In other aspects, the invention provides a method for treating a patient in need of protection from cellular death, including acute and chronic injuries to various organs or tissues, such as the brain, spinal cord, and kidney, among others. In some embodiments, the patient is in need of treatment for an ischemic organ injury or a traumatic organ injury. The method generally comprises administering an effective amount of the composition described herein to a patient in need.

In various embodiments, the patient is in need of neuroprotection. In some embodiments patient is at risk of ischemia reperfusion injury. Ischemia reperfusion injury is the tissue damage caused when blood supply returns to tissue after a period of ischemia or lack of oxygen. The absence of oxygen and nutrients from blood during the ischemic period creates a condition in which the restoration of circulation results in inflammation and oxidative damage through the induction of oxidative stress.

For example, cerebral hypoxia-ischemia (or "stroke") of sufficient duration to deplete high energy reserves in neural cells initiates a cascade of events over the hours to days of reperfusion that culminates in extensive death, both necrotic and apoptotic. These events include the generation of ROS and oxidative damage to cells, release of inflammatory mediators and initiation of prolonged inflammatory reactions, and ongoing apoptosis that can continue for weeks to months.

Thus, in some embodiments the patient is experiencing stroke. Stroke is a major cause of morbidity and mortality through all stages of the life cycle, including for infants born prematurely, for children in intensive care units, and for elderly with cerebral vascular accidents. In some embodiments, the stroke is ischemic stroke. However, the invention also finds use for treating hemorrhagic stroke as well as neonatal stroke. In some embodiments, the subject has or is at risk of Hypoxic-ischemic encephalopathy (HIE), which is a type of newborn brain damage caused by oxygen deprivation and limited blood flow. Infants and children who survive HIE demonstrate lifelong neurologic handicaps, including cerebral palsy, mental retardation, epilepsy, and learning disabilities. Vannucci, R. C. (2000), Hypoxic-ischemic encephalopathy, American Journal of Perinatology 17(3): 113-120. Cerebral hypoxia-ischemia commonly occurs in critically ill children, most notably in association with cardiopulmonary arrest.

The compositions described herein can be administered after stroke onset to provide neuroprotection, that is, inhibit cellular processes leading to cell death. The physical and chemical properties of the emulsions allow them to be effective, even when delivered later than desired after stroke onset. For example, in various embodiments, the patient is administered the composition within about 1 to about 24 hours of stroke onset. For example, in some embodiments, the composition is administered after about 6 hours of stroke onset, or after about 8 hours of stroke onset, or after about 10 hours of stroke onset, or after about 12 hours of stroke onset, or after about 15 hours of stroke onset. In some embodiments, the composition is administered after about 10 hours of stroke onset, but within 24 hours of stroke onset. The composition can prevent substantial cellular death, despite delay in emergency treatment. In some embodiments, the patient is administered the composition within about 2 hours of stroke onset or within about 4 hours of stroke onset, which provides substantial protection from cell damage and/or death.

The compositions are compatible for treating both ischemic and hemorrhagic stroke, and thus can be administered by emergency personnel, that is prior to brain imaging to detect or visualize the thrombus or potential hemorrhage. In the absence of hemorrhage, the patient may receive thrombolytic therapy to dissolve the clot (e.g., t-PA). t-PA catalyzes the conversion of plasminogen to plasmin, the major enzyme responsible for clot breakdown. t-PA is conventionally administered to a stroke victim within about the first 4.5 h after a stroke occurs. In some embodiments, the patient receives such thrombolytic therapy after about 4.5 hours from stroke onset, or after about 6 hours from stroke onset, or after about 8 hours after stroke onset, increasing thrombolytic therapeutic window by delivering t-PA together with DG emulsions. By administering the emulsion compositions as soon as possible in the emergency setting, more time can be obtained to determine whether thrombolytic therapy is appropriate. Thrombolytic therapy cannot be administered for patients experiencing hemorrhagic stroke, since the therapy would exacerbate bleeding.

In still other embodiments, a thrombectomy is performed. Thrombectomy is the interventional procedure of removing a blood clot (thrombus) from a blood vessel. It is commonly performed in the cerebral arteries (interventional neuroradiology). The compositions described herein can expand the window where thrombectomy is successful. For example, the thrombectomy may be performed after about 10 hours from stroke onset, or after about 12 hours from stroke onset. In some embodiments, thrombectomy is performed after about 18 hours or after about 24 hours of stroke onset.

In some embodiments, the patient may receive from 1 to 5 doses of the composition within the first 24 hours, with at least one dose prior to thrombolytic therapy or thrombectomy, and at least one dose after thrombolytic therapy or thrombectomy.

The composition is generally delivered parenterally, such as intravenously or intra-arterially. In some embodiments, the composition is delivered by intrathecal delivery. In some embodiments, the composition is administered intranasally, allowing for rapid delivery to the brain. In some embodiments, the composition is administered by intra-arterial delivery selectively to the previously hypoperfused brain.

In some embodiments, in the context of ischemic stroke, the subject may receive a dose of the DG emulsions as soon as possible after the onset of stroke, and generally within about 24 hours, or with about 15 hours, or within about 12 hours, or within about 10 hours, or within about 8 hours, or within about 6 hours of the onset of stroke. The patient may receive subsequent doses over the following days or weeks, to aid recovery. For example, the patient may receive at least 4 administrations of the stable DG emulsions, or may receive at least 8 administrations of the stable DG emulsions. In some embodiments, the patient receives from 1 to 10 or from 1 to 4 administrations over one week to one month following stroke to aid recovery.

In other embodiments, the patient is suffering from or at risk of traumatic brain injury (TBI). Traumatic brain injury usually results from a violent blow or jolt to the head or body. An object that penetrates brain tissue, such as a bullet or shattered piece of skull, also can cause traumatic brain injury. Mild traumatic brain injury may affect brain cells temporarily. More-serious traumatic brain injury can result in bruising, torn tissues, bleeding and other physical damage to the brain. These injuries can result in long-term complications or death. In some embodiments, the patient is administered the composition within 1 to 5 hours of brain injury, or from 1 to 2 hours of brain injury, to reduce long term tissue damage from TBI. In some embodiments, the patient is administered the composition within about 12 hours of brain injury, or within about 24 hours of brain injury. In some embodiments, the patient receives at least 4 administrations of the stable DG emulsions, or may receive at least 8 administrations of the stable DG emulsions. In some embodiments, after the initial administration, the patient is administered the composition at least 4 times or at least 10 times with frequencies ranging from about once every 4 hours to once per week to aid recovery.

In still other embodiments, the patient is suffering from post-traumatic stress disorder (PTSD). PTSD is a serious condition that develops after a person has experienced or witnessed a traumatic or terrifying event in which serious physical harm occurred or was threatened. PTSD is a lasting consequence of traumatic ordeals that cause intense fear, helplessness, or horror, such as a sexual or physical assault, the unexpected death of a loved one, an accident, war, or a natural disaster. In various embodiments, the compositions described herein provide therapeutic value for PTSD. In some embodiments, the patient is administered the composition at least once per week for a period of time to facilitate recovery.

The invention provides use for protecting other organs or tissues, including spinal cord injury (SCI). In such embodiments, the patient may be administered the composition within about 24 hours of injury, or within about 15 hours of injury, or within about 12 hours of injury, or within about 6 hours of injury, or within about 2 hours of injury, or within about 1 hour of injury. In some embodiments, the patient is administered the composition at least once per day or once per week after the initial administration. In some embodiments, the patient receives at least 4 administrations of stable DG emulsions, or may receive at least 8 administrations of stable DG emulsions. In some embodiments, after the initial administration, the patient is administered the composition at frequencies ranging from once every 4 hours to once per week (e.g., for at least four weeks) to aid recovery.

Further, in some embodiments, the patient is the recipient of an organ transplant, such as liver, kidney, heart, or lung. In some embodiments, the patient is administered the composition during the perioperative period (e.g., within about 24 hours prior to transplant surgery, and/or within about 24 hours after transplant surgery). In some embodiments, the patient receives at least 4 administrations of stable DG emulsions, or may receive at least 8 administrations of stable DG emulsions. In some embodiments, after the initial administration, the patient is administered the composition at frequencies ranging from about once every four hours to about once per week to aid recovery.

In some embodiments, the patient has acute organ failure, such as acute renal, liver, or heart failure. In some embodiments, the patient is administered the composition from 1 to 10 times or from 1 to 4 times with a frequency ranging from about once every 4 hours to once per week to reduce organ damage and/or decline.

In some embodiments, the patient is suffering from a neurodegenerative disease, such ALS, multiple sclerosis, Parkinson's disease, Alzheimer's disease, and Huntington's disease. For example, the patient is administered the composition at least once per week to slow disease progression, and/or is administered the composition upon disease relapse (e.g., in the case of MS) to reduce the severity and duration of the relapse and/or slow disease progression.

In these and other embodiments, the patient in need of neuroprotection may in addition, or in some embodiments alternatively, receive oral supplementation with n-3 fatty acids, which can optionally be in the form of DGs or n-3 TGs. Oral supplementation can be administered at least once daily and up to three times daily. Oral supplementation can be provided for one or more weeks or months as needed to support recovery from an acute event, or may be administered indefinitely to aid recovery and prevent relapse or reoccurrence of the condition. In some embodiments, oral supplementation is with n-3 DG oil, which can be administered in the form of capsules. In some embodiments, oral supplementation is dietary, for example, by providing n-3 DG oil as a component of a food product. In some embodiments, the oral supplementation is with n-3 DG emulsions.

The patient in need of neuroprotection may further receive therapy with one or more neuroprotectants, e.g., as co-therapy. Exemplary neuroprotectants include glutamate antagonists. Exemplary neuroprotectants include 17β-Estradiol, ginsenosides, progesterone, simvastatin, and memantine. These therapies can provide synergistic protection from brain injuries, along with n-3 DG emulsion therapy as described herein and/or with n-3 DG oral supplementation.

As used herein, the term "about" means ±10% of the associated numerical value.

Other aspects and embodiments of the invention will be apparent from the following examples.

### EXAMPLES

Omega-3 (n-3) fatty acids (FAs) are candidates for acute neuroprotection after stroke. A number of biological mechanisms may be affected by n-3 FAs, including (i) decrease in generation of mitochondrial reactive oxygen species (ROS); (ii) preservation of mitochondrial Ca²⁺ uptake and homeostasis; (iii) modulation of receptor-mediated signal transduction and inhibition of apoptotic pathways; (iv) increase in potent n-3 FA-derived resolvins and protectins, and (v) decrease in inflammatory responses. Working separately or synergistically, these mechanisms can contribute to n-3 FA neuroprotection in ischemic injury, decreasing cell death while accelerating repair processes.

However, to be effective for neuroprotection, adequate levels of n-3 FAs must be quickly delivered to cells at risk of cell death or injury, and thus must be delivered in a manner to effectively cross the blood-brain barrier (BBB). Nanoparticle uptake by the BBB can be through two major endocytic mechanisms, clathrin- and caveolin-mediated endocytosis. Emulsion nanoparticles with a diameter of 200 nm or less should more efficiently cross the BBB by these endocytic processes. Further, increasing the levels of n-3 FAs (in relation to PC emulsifier, for example) in small particle size emulsions may further enhance direct delivery of n-3 FAs to the brain, as well as other tissues.

This disclosure provides shelf-stable compositions and methods for acute delivery of n-3 fatty acids for treatment of ischemic stroke, traumatic brain injury and other acute organ injuries as detailed elsewhere in this application. Specifically, the following experiments provide compositions and methods to achieve stable omega-3 diglyceride (DG) oil-in-water emulsions for their use as acute therapy to treat and/or prevent organ injuries.

### Preparation and Characterization of n-3 Diglyceride Emulsions

DG emulsion formulations were developed to prepare stable emulsions with a small particle size as well as increased n-3 FA payload. As detailed in Table 1, stable DG emulsions were prepared by mixing solubilized egg yolk phosphatidylcholine (PC) with DG oils. DG oils contain at least 90% of FAs as DHA and/or EPA. Oils were prepared that differ in n-3 FA compositions - pure DG-DHA, pure DG-EPA or a mixture of DHA and EPA. DG emulsions were also prepared containing n-6 AA. Each oil was analyzed by thin layer chromatography (TLC), to determine the purity and integrity of the samples. TG emulsions were also prepared with the same process using soy oil or emulsions containing fish oil of triglycerides only containing DHA (Tri-DHA) as their fatty acid.

**Table 1: Lipid Emulsion Protocol**

| **Component** | **Amount** |
|---|---|
| n-3 DG oil or triglyceride oil | 10% to 25% by wt. of composition |
| LIPOID E80 -- phospholipid | 0.6 to 1.2% by wt. (wt. of composition before DG addition) |
| Glycerin | 2.25% by wt. (wt. of composition before DG addition) |
| Sodium oleate | 0.03% by wt. (wt. of composition before DG addition) |
| Water with 0.25 mM EDTA (pH 9.5) | To final volume |

| **Preparation** | |
|---|---|
| 1. Vortex water and glycerin at 60°C | |
| 2. Add Lipoid E80 and sodium oleate and stir moderately (e.g., 2 minutes) using gentle vortex | |
| 3. Add n-3 DG oil to the aqueous phase at 60°C | |
| 4. Mix with Homogenizer (e.g., Fisher Scientific 850 Homogenizer) for 3 min. keeping the temperature at 60°C (pre-emulsion) | |
| 5. Pass the pre-emulsion through a Microfluidizer (LV1) (high shear fluid processor) 3-5 times at 965 Bar (equal to 14000-psi) pressure at 60°C or other high pressure homogenizers for larger emulsion volumes | |
| 6. Filter emulsion using 0.45µm filter (Millipore) or use rotary autoclave for final emulsion sterilization | |
| 7. Store under Argon | |

This emulsion preparation protocol involves mixing H₂O (containing 0.25 mM EDTA) and glycerin at 60°C. Next, PC (Lipoid E80) and sodium oleate are stirred moderately for 2 min using very gentle vortex. DG oil is added to the aqueous phase at 60°C. This pre-emulsion is then mixed with a homogenizer for 3 min at 60°C. The final step involves the processing of the pre-emulsion through a high shear fluid processor (Microfluidizer, LV1 model), 3-5 times at 965-bar pressure (equal to 14000-psi) at 60°C. Following this method, we obtained a volume of up to 8 ml for each procedure. For higher volumes, higher pressure can be used. The appearance of the emulsions was as a white/milky liquid. The emulsions were stored under argon at 4°C for over 14 months after preparation. Using PC emulsifier, this process was used to successfully prepare stable 10% emulsions (10g DG/100ml) as well as stable 20% and 24% emulsions (20g DG/100ml and 24g DG/100ml). As detailed below, stable emulsions with small particle size were also obtained using 0.6-0.8 wt% PC emulsifier, and 10% and 20% DG oil. Emulsions with higher than 20% DG oil in particular have the potential to significantly improve n-3 FA payload.

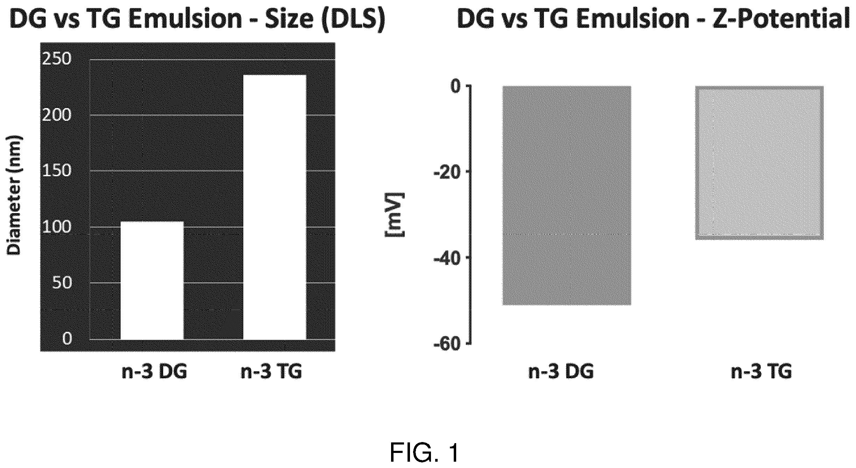

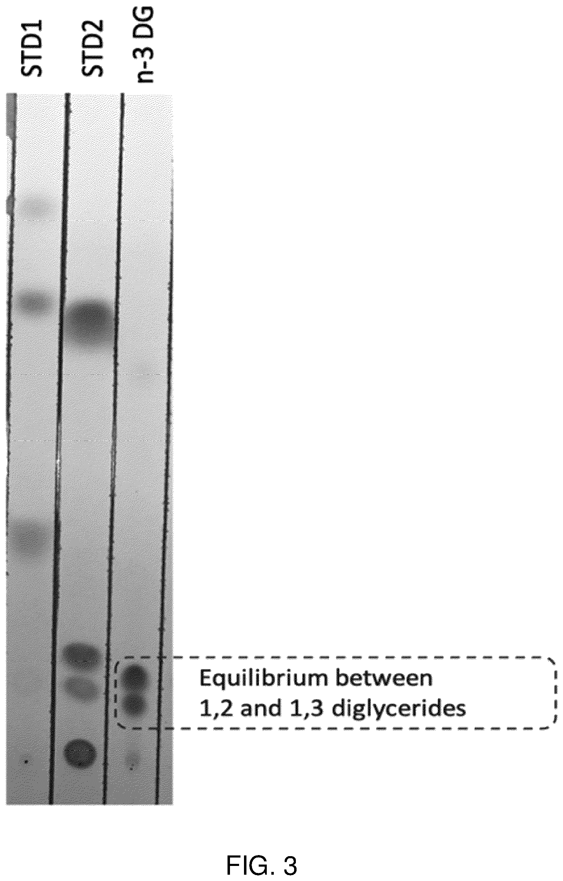

Particle size and polydispersity index (PDI) were evaluated by dynamic laser scattering (DLS). Data are analyzed in terms of composition, mean and homogeneity of the particle distribution. A representative DG emulsion is shown in FIG. 1 (left), where n-3 DG had a particle size substantially less than 200 nm (~110 nm), while TG emulsions were much larger, around 240 nm in this example. We also analyzed zeta potential, showing high repulsive forces for DG emulsions (which were substantially higher than TG), and which results in a more stable system (FIG. 1, right). The DG oil used contained about equal amounts of 1,2 and 1,3 DGs (based on the fatty acid positions) as shown by TLC (FIG. 3).

After 14 months, the same samples were analyzed, and there were no changes in PDI and mean size values, demonstrating high stability of the DG emulsions over this time period.

We hypothesized that DGs may contribute to their own emulsification and, because of the greater hydrophilicity, DGs may also incorporate more into biological membranes than TGs. To test this, we compared the effects of fatty acids delivered with either DG or TG as carriers on models of biological membrane interactions. We evaluated the solubility of DGs and TGs in the phosphatidylcholine (PC) membrane systems, and studied how DGs or TGs incorporate into the lipid mixture and possibly change the properties of PC membranes by NMR analysis. We found that omega-3 DGs have much higher incorporation into model membranes compared to omega-3 TGs, which likely contributes to their multiple and superior biological effects compared to TGs. Importantly, these results suggest that n-3 DGs in part facilitate their own emulsification together with PC, in marked contrast to TGs.

Based on these results, we then decided to lower the amounts of PL emulsifiers, from 1.2%, as in our initial preparation, to 0.8-0.6% by weight of emulsifiers (i.e., by weight of the composition prior to addition of DG oil). This was to demonstrate whether DGs are stable even with lower amount of PL. By visual inspection, the DG emulsions showed no oil droplets on the surface, while oil droplets were observed with the TG emulsion. These observations are consistent with the hypothesis that DGs work as emulsifier themselves, and therefore, stable emulsions with a higher percent of DG oil appear feasible. We propose that DG emulsions at least as high as 25 wt.% are feasible. Further, when lowering PL, the same small particle size and PDI equal or less than 0.220 is observed. Importantly, by lowering the amount of PL in the emulsions, the n-3 FA payload can be increased per dose, which can lead to critical improvements in neuroprotection.

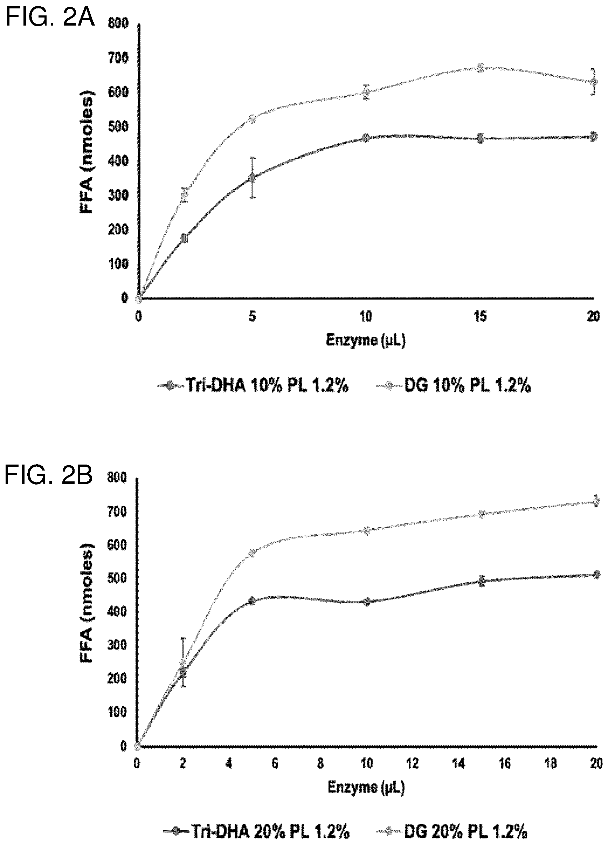

To establish that rapid hydrolysis facilitates clearance of DG emulsions, in vitro lipolysis studies were performed. FA release was assessed by lipoprotein lipase (LpL)-mediated hydrolysis of n-3 DG vs n-3 TG. The activity of purified LpL was 300-400 U/mg protein. LpL was diluted 1:20 in 0.9% NaCl at pH 8.6, immediately prior to incubation with emulsions. Experiments were performed with increasing amounts of LpL (0-20 µL of 1:20 dilution) over a fixed time (30 min). We observed that n-3 DG emulsions (both 10% and 20% emulsions) had more efficient hydrolysis compared to n-3 TG (see representative experiment, FIG. 2 A,B). These results highlight that DG structure facilitates the emulsion conversion to remnant-like particles in vivo, contributing to a faster release and uptake of n-3 FAs. Higher percentages of n-3 FAs in the emulsions can deliver higher levels of these agents to cells and tissues (compare FFA released in FIG. 2A with 2B).

Cells internalize substantial amounts of n-3 TGs via adsorptive endocytosis pathways not involving conventional cell receptors [31, 35, 36]; while mechanisms for n-6 TG uptake involve both apoE- and LDL receptor (LDLr)-dependent pathways [31, 37-39]. Differences in TG composition, particle size and hydrophilicity might explain, in part, distinct uptake processes [34, 39, 40]. The capability of whole DG particles to cross the blood-brain barrier (BBB) might depend on their physical-chemical properties as well as on specific transporters. This disclosure anticipates that increases in disorder dynamics at PL surfaces of DG emulsions as well as a small particle size will facilitate more rapid and greater in vitro uptake of n-3 FAs, in part, via "non-classical" pathways.

### Animal Models of Neuroprotection

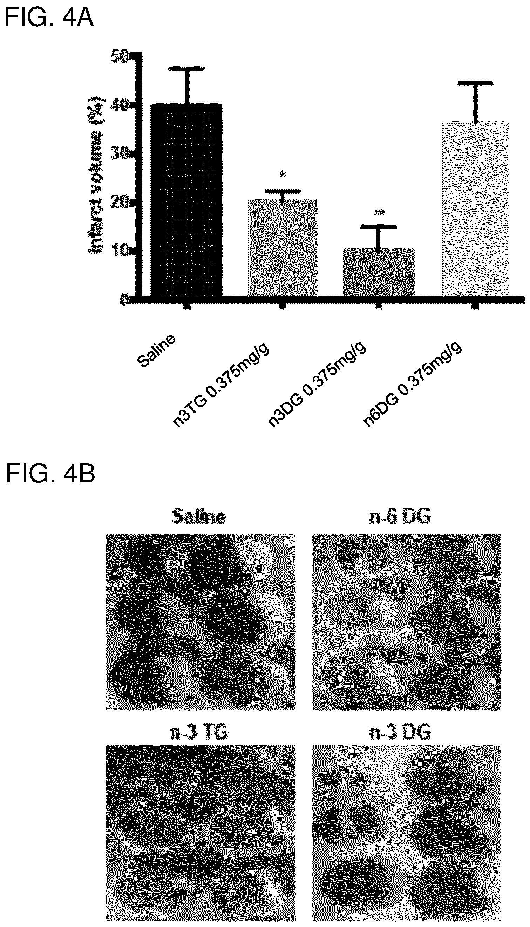

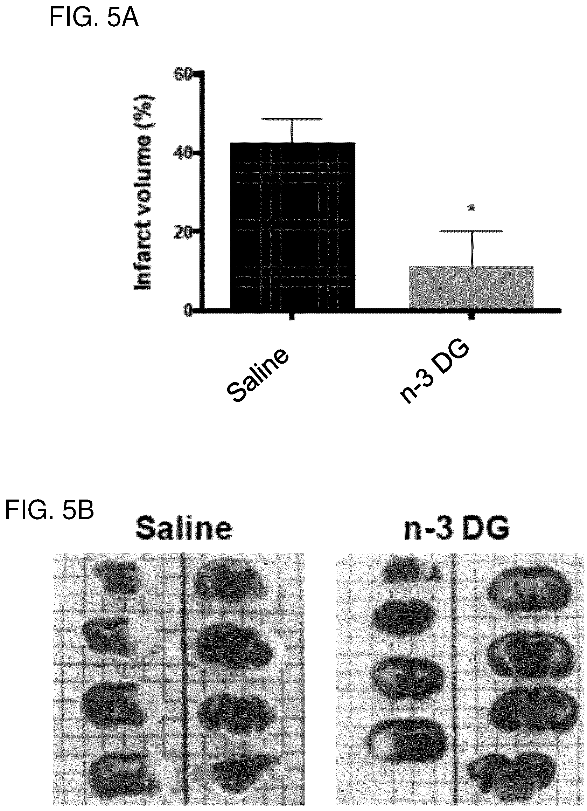

Initial exploratory data showed that neonatal mice treated with n-3 DG emulsions (containing >90% of total FAs as EPA and DHA, ~10 wt.% DG oil) exhibited significant reduction in cerebral infarct volumes at 24 hours after ischemic injury, and that n-3 DG emulsion was far more effective than n-3 TG emulsion. FIG. 4(A, B). n-6 DG treatment did not exert neuroprotection after ischemic injury. We next determined whether n-3 DG emulsions protect the brain against ischemia in an adult mouse model for stroke (C57B1/6 strain, 10-14 weeks old), using 60 min transient right middle cerebral artery occlusion (MCAo). Adult mice treated with n-3 DG emulsion (~10 wt.% DG oil) immediately after MCAo and at the beginning of reperfusion, had significantly smaller infarcts than control mice. FIG. 5(A, B). Mice treated with DG emulsion by acute bolus injection showed no adverse effects, reporting no "signaling shock" due to over-activation of DG downstream pathways.

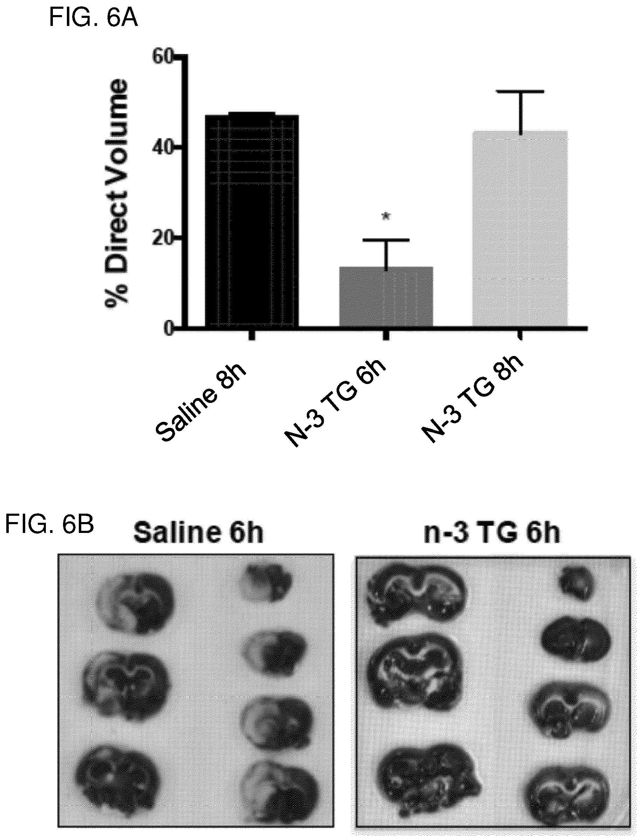

In mice, it was reported that n-3 TG emulsions possess a therapeutic time window of 2 hours after stroke [23]. However, we now find that in an adult rat model of stroke (transient right MCAo), i.v. injection of n-3 TG emulsions (10 wt.% TG oil) up to 6 hours after ischemia significantly reduced infarct volumes, suggesting longer time windows for these agents in larger mammalian species. FIG. 6(A, B).

In summary, our exploratory data highlight the potential of n-3 FAs delivered in DG emulsion to be strong neuroprotectants, providing increased efficacy over n-3 TGs in our rodent stroke models, and potentially providing for long therapeutic windows after acute injury.

We also studied the neuroprotective effects afforded by n-3 DG emulsions (DG-DHA, DG-EPA, DG-EPA+DHA) compared to n-6 DG (AA). Emulsions contained ~10 wt.% DG oils, and >90% fatty acids were n-3 FAs. Data show that neonatal mice treated with the DG emulsions, made with individual fatty acids (DHA or EPA) or with DG-DHA+EPA, exhibited significant reduction in cerebral infarct volumes when administered immediately after ischemic injury. In sharp contrast, n-6 DG with AA treatment did not exert neuroprotection after ischemic injury (FIG. 7). The % infarct volume was even lower with the DHA+EPA DG emulsions, as compared to DG emulsions with DHA and EPA alone. This improvement, demonstrated for DG emulsions containing both DHA and EPA (containing >90% of fatty acids, 10 wt.% DG oil), and having a small particle size and highly negative zeta-potential as shown herein, can provide for substantial therapeutic benefit over even our initial DG preparations. Increasing the levels of DG oils in these emulsions will likely provide even further therapeutic improvements by increasing the amount of n-3 FAs delivered in acute fashion.

### Discussion

Stroke is the leading cause of long-term disability in the United States and the 5th leading cause of death. To date, t-PA remains the only FDA-approved drug for acute ischemic stroke treatment; however, its use is limited by a narrow 3 to 4.5 h time window [2-4]. Studies presented here suggest that n-3 FAs act as bioactive unsaturated lipids with pleiotropic effects, and show neuroprotective properties in animal models of stroke. n-3 FAs injected acutely as TG emulsion can provide neuroprotection after ischemic brain injury. n-3 TG emulsions, administered immediately after ischemic injury, can lead to long-term neurofunctional and histomorphological recovery of the brain. However, a far more robust neuroprotection is achieved when n-3 FAs are carried as n-3 DGs, and injected acutely as a DG lipid emulsion after ischemic brain injury. By optimizing the composition of these DG emulsions and their physical characteristics as described here, n-3 DG emulsions have the potential to provide a highly effective and shelf-stable therapeutic treatment for acute organ injuries, including but not limited to stroke.

Adequate levels of n-3 FAs make neuronal membranes more fluid and facilitate active interactions of receptors, ion channels, and protein complexes [10, 11]. The present disclosure evaluates the enhanced neuroprotection of n-3 DGs containing both EPA and DHA, administered as an i.v. lipid emulsion with small particle size and high negative zeta potential to further potentiate n-3 FA brain delivery and efficacy in ischemic stroke. Composed of a glycerol backbone and two fatty acyl groups, DGs have a small and electrically neutral polar head; this confers a pronounced cone shape and a high capacity to undergo rapid trans-bilayer movements. The biophysical properties and physiological effects of DGs are modulated by composition of their fatty acyl groups. As integral components of cellular membranes and lipid droplets, DGs can play a key role as second messengers in cellular signaling transduction [12-16]. Phospholipids have been shown to incorporate low amounts of long chain FA TG (LCT) emulsions (2.6 mole%) with a preferred orientation of carbonyl groups positioned at the aqueous-phospholipid interface [17, 18]. Very few physical studies have emphasized DG properties on membrane bilayer organization and mobility [19-22]. Experiments here show that n-3 DG emulsions had more efficient hydrolysis compared to n-3 TG (FIG. 2 A, B). These results highlight that DG structure will facilitate the emulsion conversion to remnant-like particles and free fatty acids in vivo, contributing to a faster uptake of n-3 FAs and enhancing the onset of cytoprotective effects.

Acute treatment with DHA administered as a TG emulsion, immediately after ischemic injury, significantly attenuates brain damage [5, 23]. Data demonstrate that n-3 DG emulsions show more robust neuroprotection than n-3 TG emulsions, suggesting different metabolism of DG vs TG particles, and that n-3 DGs have distinct biological properties and specifically trigger and accelerate neuroprotective pathways crucial in initial phases of stroke. This should also contribute to an extended therapeutic time window of n-3 DG emulsions. Because of the potential of DGs to affect structural and mobility dynamics in phospholipid bilayers, DG emulsions likely represent an "improved" carrier for n-3 FAs to increase their bioavailability to the brain and to accelerate their molecular actions in modulating neuroprotective pathways.

Despite a substantial number of plausible pathways whereby n-3 FAs may reduce morbidity and mortality from cardiovascular disease, clinical n-3 FA trial results using long-term supplementations are mixed and controversial [24-27]. This disclosure challenges existing clinical paradigms by providing n-3 FAs acutely after injury, e.g., ischemic brain and heart injury. The rate of n-3 FA (e.g., EPA and DHA) tissue enrichment following oral supplementation is slow and particularly low in brain. Also, free FAs should not be directly administered by parenteral routes as they may act as detergents and have toxic side effects, such as encephalopathy [29]. In accordance with this disclosure, n-3 FAs (ideally containing DHA and EPA) are incorporated into n-3 FA DGs, as a stable i.v. lipid emulsion having a small particle size and high negative zeta potential.

Mean particle size affects stability and in vivo fate of emulsions. Zeta potential, as potential charge difference between mobile particles and the layer of dispersant around them, is used as an indicator of emulsion stability. In accordance with this disclosure, it is believed that reduced mean particle size and zeta potential will enhance stability of DG vs TG emulsions by lowering separation and aggregation phenomena. n-3 DG oil (DHA/EPA ~1.3/1, w/w) was used and incorporated into emulsions. As shown in FIG. 1, smaller mean particle diameters for n-3 DG (~110 nm) were observed as compared to TG emulsions (~240 nm), which may provide for faster and higher uptake of these n-3 DG emulsions by endocytosis and delivery to the brain, as well as a higher surface to core DG ratio to enable more rapid hydrolysis. See FIG. 2(A,B). The zeta potential for TG was -35 mV, while the zeta potential of DG was -51 mV in FIG. 1. The net negative charge at the interface in both emulsions is sufficient to prevent flocculation and aggregation through strong electrostatic repulsive forces; however, the lower negativity in DG should translate into a greater stability of DG emulsions.

Optimization of DG emulsion formulations is screened by mean particle sizes, PDI as homogeneity indicator, zeta potential, and electron microscopy. Oil-water interface advantages might positively affect the interaction of the n-3 DG emulsions demonstrated herein with cellular endocytic and catabolic pathways. In fact, our studies show that DGs had substantially faster lipolysis compared to TGs. See FIG. 2(A, B).

After injection, n-3 FAs are taken up into brain more efficiently than shorter chain FAs [10]. However, no information is available regarding brain delivery of DHA or EPA injected as n-3 DG emulsions. Previous data showed after injection of radiolabeled n-3 TG emulsions a significant increase in plasma TG levels within 30 min [23], with <0.5% of particles entering brain. Significant increases in mitochondrial but not whole brain n-3 FA levels were observed [5]. Liver accounted for the highest organ uptake of n-3 TG particles (>50%) [31]. This suggests that neuroprotection observed for n-3 TG emulsion may depend on its delivery, "repackaging" and metabolism in other organs prior to its direct effects in brain. It is believed that the DG emulsions disclosed here, containing >90% of FAs as DHA and EPA, and having a small particle size less than 200 nm, will allow for faster catabolic uptake, and should have additional uptake routes, including directly to the brain. It is anticipated that, after acute administration, n-3 DGs are partially taken up by the liver and then repackaged into either free FAs or into liver-produced lipoproteins and transported directly to the brain. However, brain uptake of intact emulsion particles may also contribute for clearance of DG emulsions in vivo. It is anticipated that the DG emulsions described herein represent a more efficient carrier for n-3 FAs to brain, providing an alternative and/or additive therapeutic approach for stroke.

In accordance with this disclosure, it is anticipated that the DG emulsions described herein will increase n-3 FA clearance and incorporation into the brain compared to TG emulsions or DG emulsions having a larger particle size (e.g., greater than 200 nm) or containing only DHA or EPA. We anticipate that after ischemia, intact whole DG particles will also cross the BBB, facilitating brain uptake of n-3 FAs. We anticipate greater neuroprotection by n-3 DG emulsions described here as compared to n-3 TG emulsions and as compared to DG emulsions having a larger particle size or containing only DHA or EPA. We expect, thus, that n-3 DG emulsions described here will show a prolonged neuroprotective time window after ischemic injury, e.g, >6 hours in rats, demonstrating the superiority of these n-3 DG emulsions.

### REFERENCES

1. Benjamin EJ, et al. Heart Disease and Stroke Statistics-2018 Update: A Report from the American Heart Association. Circulation. 2018 Mar 20;137(12):e67-e492. doi: 10.1161/CIR.0000000000000558. Epub 2018 Jan 31.
2. Marshall RS. Progress in Intravenous Thrombolytic Therapy for Acute Stroke. JAMA Neurol. 2015 Aug;72(8):928-34. doi: 10.1001/jamaneurol.2015.0835.
3. Jauch EC, ET AL. American Heart Association Stroke Council; Council on Cardiovascular Nursing; Council on Peripheral Vascular Disease; Council on Clinical Cardiology. Guidelines for the early management of patients with acute ischemic stroke: a guideline for healthcare professionals from the American Heart Association/American Stroke Association. Stroke. 2013 Mar;44(3):870-947. doi: 10.1161/STR.0b013e318284056a. Epub 2013 Jan 31.
4. Brott TG, et al. Urgent therapy for stroke. Part I. Pilot study of tissue plasminogen activator administered within 90 minutes. Stroke. 1992 May;23(5):632-40.
5. Mayurasakorn K, et al. DHA but Not EPA emulsions preserve neurological and mitochondrial function after brain hypoxia-Ischemia in neonatal mice. PLoS One. 2016; 11(8): e0160870.
6. Zhang T, et al. Docosahexaenoic Acid Alleviates Oxidative Stress-Based Apoptosis Via Improving Mitochondrial Dynamics in Early Brain Injury After Subarachnoid Hemorrhage. Cell Mol Neurobiol. 2018 Oct;38(7):1413-1423. doi: 10.1007/s10571-018-0608-3. Epub 2018 Aug 6.
7. Zirpoli H, et al. NPD1 rapidly targets mitochondria-mediated apoptosis after acute injection protecting brain against ischemic injury. Exp Neurol. 2021 Jan;335:113495. doi: 10.1016/j.expneurol.2020.113495. Epub 2020 Oct 8.
8. Layé S, Nadjar A, Joffre C, Bazinet RP. Anti-Inflammatory Effects of Omega-3 Fatty Acids in the Brain: Physiological Mechanisms and Relevance to Pharmacology. Pharmacol Rev. 2018 Jan;70(1):12-38. doi: 10.1124/pr.117.014092.
9. Pu H, et al. Delayed Docosahexaenoic Acid Treatment Combined with Dietary Supplementation of Omega-3 Fatty Acids Promotes Long-Term Neurovascular Restoration After Ischemic Stroke. Transl Stroke Res. 2016 Dec;7(6):521-534. Epub 2016 Aug 27.
10. Mayurasakorn K, Williams JJ, Ten VS, Deckelbaum RJ. Docosahexaenoic acid: brain accretion and roles in neuroprotection after brain hypoxia and ischemia. Curr Opin Clin Nutr Metab Care. 2011 Mar; 14(2):158-67.
11. Bazinet RP, Laye S. Polyunsaturated fatty acids and their metabolites in brain function and disease. Nature Reviews Neuroscience. 2014;15(12):771-85.
12. Nishizuka Y. The role of protein kinase C in cell surface signal transduction and tumour promotion. Nature. 1984 Apr 19-25; 308(5961):693-8.
13. Berridge MJ, Irvine RF. Inositol trisphosphate, a novel second messenger in cellular signal transduction. Nature. 1984 Nov 22-28; 312(5992):315-21.
14. Dennis EA1, Rhee SG, Billah MM, Hannun YA. Role of phospholipase in generating lipid second messengers in signal transduction. FASEB J. 1991 Apr; 5(7):2068-77.
15. Shimada A, Ohashi K. Interfacial and Emulsifying Properties of Diacylglycerol. Food Sci. Technol. Res, 2003 9 (2); 142-147.
16. Tada N, Yoshida H. Diacylglycerol on lipid metabolism. Curr Opin Lipidol. 2003 Feb; 14(1):29-33.
17. Hamilton JA, Vural JM, Carpentier YA, Deckelbaum RJ. Incorporation of medium chain triacylglycerols into phospholipid bilayers: effect of long chain triacylglycerols, cholesterol, and cholesteryl esters. J Lipid Res. 1996 Apr; 37(4):773-82.
18. Johnson RA, Hamilton JA, Worgall TS, Deckelbaum RJ. Free fatty acids modulate intermembrane trafficking of cholesterol by increasing lipid mobilities: novel 13C NMR analyses of free cholesterol partitioning. Biochemistry. 2003 Feb 18;42(6):1637-45.
19. Epand RM. Diacylglycerols, lysolecithin, or hydrocarbons markedly alter the bilayer to hexagonal phase transition temperature of phosphatidylethanolamines. Biochemistry. 1985 Dec 3; 24(25):7092-5.
20. Das S, Rand RP. Modification by diacylglycerol of the structure and interaction of various phospholipid bilayer membranes. Biochemistry. 1986 May 20; 25(10):2882-9.
21. Yasunaga K, et al. Effects of triacylglycerol and diacylglycerol oils on blood clearance, tissue uptake, and hepatic apolipoprotein B secretion in mice. J Lipid Res. 2007 May;48(5):1108-21. Epub 2007 Feb 3.
22. Harvey K, et al. Parenteral lipid emulsions in guinea pigs differentially influence plasma and tissue levels of fatty acids, squalene, cholesterol, and phytosterols. Lipids. 2014 Aug; 49(8):777-93.
23. Williams JJ, et al. N-3 fatty acid rich triglyceride emulsions are neuroprotective after cerebral hypoxic-ischemic injury in neonatal mice. PLoS One. 2013; 8(2): e56233.
24. Mozaffarian, D. & Wu, J. H. Omega-3 fatty acids and cardiovascular disease: effects on risk factors, molecular pathways, and clinical events. J. Am. Coll. Cardiol. 2011; 58, 2047-2067.
25. Aung T, et al. Omega-3 Treatment Trialists' Collaboration. Associations of omega-3 fatty acid supplement use with cardiovascular disease risks: Metaanalysis of 10 trials involving 77 917 individuals. JAMA Cardiol. 2018 Jan 31. doi: 10.1001/jamacardio.2017.5205.
26. Abdelhamid AS, et al. Cochrane Database Syst Rev. 2018 Jul 18;7:CD003177. doi: 10.1002/14651858.CD003177.pub3. Omega-3 fatty acids for the primary and secondary prevention of cardiovascular disease.
27. Brinton EA, et al. Lipid Effects of Icosapent Ethyl in Women with Diabetes Mellitus and Persistent High Triglycerides on Statin Treatment: ANCHOR Trial Subanalysis. J Womens Health (Larchmt). 2018 Sep;27(9):1170-1176. doi: 10.1089/jwh.2017.6757. Epub 2018 Mar 27.
28. Zirpoli H, et al. Acute administration of n-3 rich triglyceride emulsions provides cardioprotection in murine models after ischemia-reperfusion. PLoS One. 2015 Jan 5;10(1):e0116274. doi: 10.1371/journal.pone.0116274. eCollection 2015.
29. Singh AK, Yoshida Y, Garvin AJ, Singh I. Effect of fatty acids and their derivatives on mitochondrial structures. J Exp Pathol. 1989; 4(1):9-15.
30. Deckelbaum RJ, et al. Medium-chain versus long-chain triacylglycerol emulsion hydrolysis by lipoprotein lipase and hepatic lipase: implications for the mechanisms of lipase action. Biochemistry. 1990 Feb 6; 29(5):1136-42.
31. Qi K, Seo T, Al-Haideri M, Worgall TS, Vogel T, Carpentier YA, Deckelbaum RJ. Omega-3 triglycerides modify blood clearance and tissue targeting pathways of lipid emulsions. Biochemistry. 2002 Mar 5; 41(9):3119-27.
32. Chang CL, et al. Lipoprotein Lipase Deficiency Impairs Bone Marrow Myelopoiesis and Reduces Circulating Monocyte Levels. Arterioscler Thromb Vasc Biol. 2018 Mar;38(3):509-519. doi: 10.1161/ATVBAHA.117.310607. Epub 2018 Jan 25.
33. Benson SP, Pleiss J. Molecular dynamics simulations of self-emulsifying drug-delivery systems (SEDDS): influence of excipients on droplet nanostructure and drug localization. Langmuir. 2014 Jul 22; 30(28):8471-80.
34. Qi K, Al-Haideri M, Seo T, Carpentier YA, Deckelbaum RJ. Effects of particle size on blood clearance and tissue uptake of lipid emulsions with different triglyceride compositions. JPEN J Parenter Enteral Nutr. 2003 Jan-Feb;27(1):58-64.
35. Murray-Taylor FM, Ho YY, Densupsoontorn N, Chang CL, Deckelbaum RJ, Seo T. n-3 but not n-6 lipid particle uptake requires cell surface anchoring. Biochem Biophys Res Commun. 2010 Feb 5;392(2):135-9. doi: 10.1016/j.bbrc.2009.12.164.
36. Densupsoontorn N, et al. CD36 and proteoglycan-mediated pathways for (n-3) fatty acid enriched triglyceride-rich particle blood clearance in mouse models in vivo and in peritoneal macrophages in vitro. J Nutr. 2008 Feb;138(2):257-61.
37. Al-Haideri M, et al. Heparan sulfate proteoglycan-mediated uptake of apolipoprotein E-triglyceride-rich lipoprotein particles: a major pathway at physiological particle concentrations. Biochemistry. 1997;36(42):12766-72.
38. Schwiegelshohn B, et al. Effects of apoprotein E on intracellular metabolism of model triglyceride-rich particles are distinct from effects on cell particle uptake. J Biol Chem. 1995;270(4):1761-9.
39. Granot E, et al. Effects of particle-size on cell uptake of model triglyceride-rich particles with and without apoprotein E. Biochemistry. 1994:33(50): 15190-7.
40. Oliveira FL, Rumsey SC, Schlotzer E, Hansen I, Carpentier YA, Deckelbaum RJ. Triglyceride hydrolysis of soy oil vs fish oil emulsions. JPEN J Parenter Enteral Nutr. 1997;21 (4):224-9.
41. Deckelbaum RJ, Ramakrishnan R, Eisenberg S, Olivecrona T, Bengtsson-Olivecrona G. Triacylglycerol and phospholipid hydrolysis in human plasma lipoproteins: role of lipoprotein and hepatic lipase. Biochemistry. 1992 Sep 15;31(36):8544-51.
42. Weksler B, Romero IA, Couraud PO. The hCMEC/D3 cell line as a model of the human blood brain barrier. Fluids Barriers CNS. 2013 Mar 26;10(1):16. doi: 10.1186/2045-8118-10-16.
43. Seo T, Al-Haideri M, Treskova E, Worgall TS, Kako Y, Goldberg IJ, Deckelbaum RJ. Lipoprotein lipasemediated selective uptake from low density lipoprotein requires cell surface proteoglycans and is independent of scavenger receptor class B type 1. J Biol Chem. 2000 Sep 29;275(39):30355-62.
44. Cattelotte J, André P, Ouellet M, Bourasset F, Scherrmann JM, Cisternino S. In situ mouse carotid perfusion model: glucose and cholesterol transport in the eye and brain. J Cereb Blood Flow Metab. 2008 Aug;28(8):1449-59. doi: 10.1038/jcbfm.2008.34. Epub 2008 Apr.
45. Thomas A, Detilleux J, Flecknell P, Sandersen C. Impact of Stroke Therapy Academic Industry Roundtable (STAIR) Guidelines on Peri-Anesthesia Care for Rat Models of Stroke: A Meta-Analysis Comparing the Years 2005 and 2015. PLoS One. 2017 Jan 25;12(1): e0170243. doi: 10.1371/journal.pone.0170243. eCollection 2017.
46. Menzies SA, Hoff JT, Betz AL. Middle cerebral artery occlusion in rats: a neurological and pathological evaluation of a reproducible model. Neurosurgery. 1992 Jul.
47. Yoon JS, Jo D, Lee HS, Yoo SW, Lee TY, Hwang WS, Choi JM, Kim E, Kim SS, Suh-Kim H. Spatiotemporal Protein Atlas of Cell Death-Related Molecules in the Rat MCAO Stroke Model. Exp Neurobiol. 2018 Aug;27(4):287-298. doi: 10.5607/en.2018.27.4.287. Epub 2018 Aug 16.
48. Nijboer CH, Groenendaal F, Kavelaars A, Hagberg HH, van Bel F, Heijnen CJ. Gender-specific neuroprotection by 2-iminobiotin after hypoxia-ischemia in the neonatal rat via a nitric oxide independent pathway. J Cereb Blood Flow Metab. 2007;27(2):282-92.
49. Davis JB and Maher P (1994) Protein kinase C activation inhibits glutamate-induced cytotoxicity in a neuronal cell line. Brain Res 652(1): 169-173.
50. Sassa S, Sugita O, Galbraith RA, Kappas A. Drug metabolism by the human hepatoma cell, HepG2. Biochem Biophys Res Commun. 1987; 143:52-57. doi: 10.1016/0006-291X (87)90628-0.

## Claims

1. A composition suitable for intravenous or intra-arterial injection, the composition comprising a stable diglyceride (DG) oil-in-water emulsion; wherein the emulsion comprises 10% by weight of a DG oil, the esterified fatty acids of the DG oil being at least about 90% n-3 fatty acids and comprising DHA and EPA; wherein the emulsion has a mean particle size of from 110 nm to 180 nm with a polydispersity index of 0.3 or less, and a zeta potential (ZP) of about -30 mV or more negative than -30 mV;
wherein the emulsion further comprises phospholipid emulsifier, which comprises phosphatidylcholine; and
wherein the emulsion optionally further comprises oleic acid emulsifier, which is optionally present at from about 0.01% to about 0.1% by weight.

2. The composition of claim 1, wherein the emulsion comprises oleic acid or alkali metal salt thereof.

3. The composition of claim 1, wherein the particle size and polydispersity index is stable for at least one year.

4. The composition of claim 3, wherein the composition is contained in a pre-filled syringe.

5. The composition of claim 4, wherein the composition has a volume for injection of from about 1 mL to about 50 mL.

6. The composition of claim 3, wherein the composition is packaged in vials.

7. The composition of claim 6, wherein the composition has a volume of about 25 mL to about 100 mL per vial.

8. The composition of any one or claims 1 to 7, wherein the emulsions are co formulated with one or more other lipophilic agents,
optionally wherein the lipophilic agent is glibenclamide,
or wherein the lipophilic agent is a statin, which is optionally selected from atorvastatin, fluvastatin, lovastatin, simvastatin and cerivastatin, or
wherein the lipophilic agent is a neuroprotectant, which is optionally selected from 17 -Estradiol, a ginsenoside, progesterone, simvastatin, and memantine, or
wherein the lipophilic agent is a metabolite of EPA, DHA, or DPA.

9. The composition of claim 8 wherein the metabolite is a resolvin or protectin, and optionally wherein the emulsions comprise NPD1.

10. A composition of any one of claims 1 to 9 for use in a method for treating a patient in need of protection from cellular death, comprising, administering an effective amount of the composition to said patient in need.

11. The composition for the use of claim 10, wherein the patient is in need of neuroprotection, and/or wherein the patient is at risk of ischemia reperfusion injury,
optionally wherein the patient is experiencing stroke, optionally wherein the stroke is ischemic stroke,
or wherein the patient is experiencing hemorrhagic stroke,
or wherein the patient is experiencing neonatal stroke.

12. The composition for the use of claim 11, wherein the subject has or is at risk for Hypoxic- Ischemic Encephalopathy (HIE).

13. The composition for the use of any one of claims 10 to 12, wherein the patient is administered the composition within about 20 hours of the onset of injury,
optionally wherein the patient is administered the composition after about 6 hours of stroke onset, or after about 8 hours of stroke onset, or after about 10 hours of stroke onset, or after about 12 hours from stroke onset, or
wherein the patient is administered the composition within about 2 hours of stroke onset, or within about 6 hours of stroke onset,
and/or wherein the patient is administered the composition prior to brain imaging, optionally wherein the patient subsequently receives thrombolytic therapy, and wherein the patient receives thrombolytic therapy after about 4.5 hours from stroke onset, or after about 6 hours from stroke onset, or after about 8 hours from stroke onset, or
wherein a thrombectomy is performed, optionally wherein the thrombectomy is performed after about 10 hours from stroke onset, or after about 12 hours from stroke onset, or up to 24 hours after stroke onset,
and/or wherein the patient receives from 1 to 5 doses of the composition within 24 hours, optionally with at least one dose prior to thrombolytic therapy or thrombectomy, and at least one dose after thrombolytic therapy or thrombectomy,
and/or wherein the composition is delivered intravenously or intra-arterially, optionally wherein the composition is administered by intra-arterial delivery selectively to the previously hypoperfused brain, or wherein the composition is delivered intrathecally, or wherein the composition is delivered by intragastric or intraduodenal tube.

14. The composition for the use of claim 10, wherein the patient is suffering from or at risk of traumatic brain injury, optionally wherein the patient is administered the composition within about 1 hour of brain injury, or within about 2 hours of brain injury, or with about 12 hours of brain injury, or within about 24 hours of brain injury, optionally wherein the patient is administered the composition from 1 to 10 times, with frequencies ranging from about once every four hours to about once per week,
or wherein the patient is suffering from post-traumatic stress disorder (PTSD), optionally wherein the patient is administered the composition about once per week,
or wherein the patient is suffering a spinal cord injury, optionally wherein the patient is administered the composition within about 1 hour or within about 2 hours of injury, and/or wherein the patient is administered the composition at least once per week for at least four weeks,
or wherein the patient is suffering acute organ injury,
or wherein the patient is suffering from a neurodegenerative disease, optionally wherein the neurodegenerative disease is ALS, multiple sclerosis, Parkinson's disease, Alzheimer's disease, or Huntington's disease, and/or wherein the patient is administered the composition at least once per week, and/or is administered during periods of relapse.

15. The composition for the use of claim 10, wherein the patient further receives oral supplementation therapy with n-3 FAs, optionally as DGs or TGs.

## Patentansprüche

1. Zusammensetzung, die zur intravenösen oder intraarteriellen Injektion geeignet ist, wobei die Zusammensetzung eine stabile Diglycerid (DG)-Öl-in-Wasser-Emulsion umfasst; wobei die Emulsion 10 Gew.-% eines DG-Öls umfasst, wobei die veresterten Fettsäuren des DG-Öls mindestens ungefähr 90 % n-3-Fettsäuren sind und DHA und EPA umfassen; wobei die Emulsion eine mittlere Partikelgröße von 110 nm bis 180 nm mit einem Polydispersitätsindex von 0,3 oder weniger und ein Zetapotential (ZP) von ungefähr -30 mV oder negativer als -30 mV aufweist;
wobei die Emulsion ferner einen Phospholipidemulgator umfasst, der Phosphatidylcholin umfasst; und
wobei die Emulsion wahlweise ferner einen Ölsäureemulgator umfasst, der wahlweise in einer Menge von von ungefähr 0,01 Gew.-% bis zu ungefähr 0,1 Gew.-% vorhanden ist.

2. Zusammensetzung nach Anspruch 1, wobei die Emulsion Ölsäure oder ein Alkalimetallsalz davon umfasst.

3. Zusammensetzung nach Anspruch 1, wobei die Partikelgröße und der Polydispersitätsindex über mindestens ein Jahr stabil sind.

4. Zusammensetzung nach Anspruch 3, wobei die Zusammensetzung in einer vorgefüllten Spritze enthalten ist.

5. Zusammensetzung nach Anspruch 4, wobei die Zusammensetzung ein Injektionsvolumen von ungefähr 1 ml bis ungefähr 50 ml aufweist.

6. Zusammensetzung nach Anspruch 3, wobei die Zusammensetzung in Fläschchen verpackt ist.

7. Zusammensetzung nach Anspruch 6, wobei die Zusammensetzung ein Volumen von ungefähr 25 ml bis ungefähr 100 ml pro Fläschchen aufweist.

8. Zusammensetzung nach einem der Ansprüche 1 bis 7, wobei die Emulsionen mit einem oder mehreren anderen lipophilen Mitteln koformuliert sind,
wahlweise, wobei das lipophile Mittel Glibenclamid ist,
oder wobei das lipophile Mittel ein Statin ist, das wahlweise ausgewählt ist aus Atorvastatin, Fluvastatin, Lovastatin, Simvastatin und Cerivastatin, oder
wobei das lipophile Mittel ein Neuroprotektivum ist, das wahlweise ausgewählt ist aus 17-Estradiol, einem Ginsenosid, Progesteron, Simvastatin und Memantin, oder
wobei das lipophile Mittel ein Metabolit von EPA, DHA oder DPA ist.

9. Zusammensetzung nach Anspruch 8, wobei der Metabolit ein Resolvin oder Protectin ist und wobei die Emulsionen wahlweise NPD1 umfassen.

10. Zusammensetzung nach einem der Ansprüche 1 bis 9 zur Verwendung in einem Verfahren zur Behandlung eines Patienten, der Schutz vor Zelltod benötigt, umfassend die Verabreichung einer wirksamen Menge der Zusammensetzung an den Patienten, der dies benötigt.

11. Zusammensetzung zur Verwendung nach Anspruch 10, wobei der Patient neuroprotektive Schutzmaßnahmen benötigt, und/oder wobei der Patient dem Risiko einer Ischämie-Reperfusionsschädigung ausgesetzt ist,
wahlweise, wobei der Patient einen Schlaganfall erleidet, wahlweise, wobei es sich bei dem Schlaganfall um einen ischämischen Schlaganfall handelt,
oder wobei der Patient einen hämorrhagischen Schlaganfall erleidet,
oder wobei der Patient einen neonatalen Schlaganfall erleidet.

12. Zusammensetzung zur Verwendung nach Anspruch 11, wobei die Person eine hypoxisch-ischämische Enzephalopathie (HIE) aufweist oder dem Risiko einer solchen ausgesetzt ist.

13. Zusammensetzung zur Verwendung nach einem der Ansprüche 10 bis 12, wobei die Zusammensetzung dem Patienten innerhalb von ungefähr 20 Stunden nach Einsetzen der Verletzung verabreicht wird,
wahlweise, wobei dem Patienten die Zusammensetzung nach ungefähr 6 Stunden nach Einsetzen des Schlaganfalls oder nach ungefähr 8 Stunden nach Einsetzen des Schlaganfalls oder nach ungefähr 10 Stunden nach Einsetzen des Schlaganfalls oder nach ungefähr 12 Stunden nach Einsetzen des Schlaganfalls verabreicht wird, oder
wobei dem Patienten die Zusammensetzung innerhalb von ungefähr 2 Stunden nach Einsetzen des Schlaganfalls oder innerhalb von ungefähr 6 Stunden nach Einsetzen des Schlaganfalls verabreicht wird,
und/oder wobei dem Patienten die Zusammensetzung vor Gehirnbildgebung verabreicht wird, wahlweise, wobei der Patient darauffolgend eine thrombolytische Therapie erhält, und wobei der Patient eine thrombolytische Therapie nach ungefähr 4,5 Stunden nach Einsetzen des Schlaganfalls, oder nach ungefähr 6 Stunden nach Einsetzen des Schlaganfalls, oder nach ungefähr 8 Stunden nach Einsetzen des Schlaganfalls erhält, oder
wobei eine Thrombektomie durchgeführt wird, wahlweise, wobei die Thrombektomie nach ungefähr 10 Stunden nach Einsetzen des Schlaganfalls oder nach ungefähr 12 Stunden nach Einsetzen des Schlaganfalls oder bis zu 24 Stunden nach Einsetzen des Schlaganfalls durchgeführt wird,
und/oder wobei der Patient innerhalb von 24 Stunden 1 bis 5 Dosen der Zusammensetzung erhält, wahlweise mit mindestens einer Dosis vor der thrombolytischen Therapie oder Thrombektomie und mindestens einer Dosis nach der thrombolytischen Therapie oder Thrombektomie,
und/oder wobei die Zusammensetzung intravenös oder intraarteriell verabreicht wird, wobei die Zusammensetzung wahlweise durch intraarterielle Verabreichung selektiv an das zuvor hypoperfundierte Gehirn verabreicht wird, oder wobei die Zusammensetzung intrathekal verabreicht wird, oder wobei die Zusammensetzung durch eine intragastrische oder intraduodenale Röhre verabreicht wird.

14. Zusammensetzung zur Verwendung nach Anspruch 10, wobei der Patient an einer traumatischen Hirnverletzung leidet oder dem Risiko einer solchen ausgesetzt ist, wahlweise, wobei dem Patienten die Zusammensetzung innerhalb von ungefähr 1 Stunde nach der Hirnverletzung oder innerhalb von ungefähr 2 Stunden nach der Hirnverletzung oder innerhalb von ungefähr 12 Stunden nach der Hirnverletzung oder innerhalb von ungefähr 24 Stunden nach der Hirnverletzung verabreicht wird, wahlweise, wobei dem Patienten die Zusammensetzung 1 bis 10 Mal mit Frequenzen im Bereich von ungefähr einmal alle vier Stunden bis ungefähr einmal pro Woche verabreicht wird,
oder wobei der Patient an einer posttraumatischen Belastungsstörung (PTSD) leidet, wahlweise, wobei dem Patienten die Zusammensetzung ungefähr einmal pro Woche verabreicht wird,
oder wobei der Patient an einer Rückenmarksverletzung leidet, wahlweise, wobei dem Patienten die Zusammensetzung innerhalb von ungefähr 1 Stunde oder innerhalb von ungefähr 2 Stunden nach der Verletzung verabreicht wird, und/oder wobei dem Patienten die Zusammensetzung mindestens einmal pro Woche über mindestens vier Wochen verabreicht wird,
oder wobei der Patient an einer akuten Organverletzung leidet,
oder wobei der Patient an einer neurodegenerativen Erkrankung leidet, wahlweise, wobei die neurodegenerative Erkrankung ALS, Multiple Sklerose, Parkinson-Krankheit, Alzheimer-Krankheit oder Huntington-Krankheit ist, und/oder wobei dem Patienten die Zusammensetzung mindestens einmal pro Woche verabreicht wird und/oder während Zeiträumen eines Rückfalls verabreicht wird.

15. Zusammensetzung nach Anspruch 10, wobei der Patient ferner eine orale Supplementierungstherapie mit n-3-Fettsäuren, wahlweise als DGs oder TGs, erhält.

## Revendications

1. Composition adaptée pour une injection intraveineuse ou intra-artérielle, la composition comprenant une émulsion huile-en-eau de diglycéride (DG) stable ; dans laquelle l'émulsion comprend 10 % en poids d'une huile DG, les acides gras estérifiés de l'huile DG étant d'au moins à environ 90 % des acides gras n-3 et comprenant du DHA et de l'EPA ; dans laquelle l'émulsion présente une taille moyenne de particules de 110 nm à 180 nm avec un indice de polydispersité de 0,3 ou moins, et un potentiel zêta (ZP) d'environ -30 mV ou plus négatif que -30 mV ;
dans laquelle l'émulsion comprend en outre un émulsionnant de phospholipide, qui comprend une phosphatidylcholine ; et
dans laquelle l'émulsion comprend en outre facultativement un émulsionnant d'acide oléique, qui est facultativement présent d'environ 0,01 % à environ 0,1 % en poids.

2. Composition selon la revendication 1, dans laquelle l'émulsion comprend de l'acide oléique ou un sel de métal alcalin de celui-ci.

3. Composition selon la revendication 1, dans laquelle la taille de particules et l'indice de polydispersité sont stables pendant au moins un an.

4. Composition selon la revendication 3, dans laquelle la composition est contenue dans une seringue préremplie.

5. Composition selon la revendication 4, dans laquelle la composition présente un volume pour injection d'environ 1 ml à environ 50 ml.

6. Composition selon la revendication 3, la composition étant emballée dans des flacons.

7. Composition selon la revendication 6, la composition présentant un volume d'environ 25 ml à environ 100 ml par flacon.

8. Composition selon l'une quelconque des revendications 1 à 7, dans laquelle les émulsions sont co-formulées avec un ou plusieurs autres agents lipophiles,
facultativement dans laquelle l'agent lipophile est un glibenclamide,
ou dans laquelle l'agent lipophile est une statine, qui est facultativement sélectionnée parmi l'atorvastatine, la fluvastatine, la lovastatine, la simvastatine et la cérivastatine, ou
dans lequel l'agent lipophile est un agent neuroprotecteur, qui est facultativement sélectionné parmi le 17-oestradiol, un ginsénoside, la progestérone, la simvastatine et la mémantine, ou
dans laquelle l'agent lipophile est un métabolite d'EPA, DHA, ou DPA.

9. Composition selon la revendication 8, dans laquelle le métabolite est une résolvine ou une protectine, et facultativement dans laquelle les émulsions comprennent du NPD1.

10. Composition selon l'une quelconque des revendications 1 à 9 pour utilisation dans une méthode de traitement d'un patient ayant besoin de protection contre la mort cellulaire, comprenant l'administration d'une quantité efficace de la composition audit patient en ayant besoin.

11. Composition pour utilisation selon la revendication 10, dans laquelle le patient a besoin de neuroprotection, et/ou dans laquelle le patient présente un risque de lésion d'ischémie-reperfusion,
facultativement dans laquelle le patient subit un AVC, facultativement dans laquelle l'AVC est un AVC ischémique,
ou dans laquelle le patient subit un AVC hémorragique,
ou dans laquelle le patient subit un AVC néonatal.

12. Composition pour utilisation selon la revendication 11, dans laquelle le sujet est atteint de ou présente un risque d'encéphalopathie hypoxique-ischémique (HIE).

13. Composition pour utilisation selon l'une quelconque des revendications 10 à 12, dans laquelle le patient se voit administrer la composition dans les 20 heures environ après le début de la lésion,
facultativement dans laquelle le patient se voit administrer la composition environ 6 heures après le début de l'AVC, ou environ 8 heures après le début de l'AVC, ou environ 10 heures après le début de l'AVC, ou environ 12 heures après le début de l'AVC, ou
dans laquelle le patient se voit administrer la composition dans un délai d'environ 2 heures après le début de l'AVC, ou dans un délai d'environ 6 heures après le début de l'AVC,
et/ou dans laquelle le patient se voit administrer la composition avant l'imagerie cérébrale, facultativement dans laquelle le patient reçoit ensuite une thérapie thrombolytique, et dans laquelle le patient reçoit une thérapie thrombolytique environ 4,5 heures après le début de l'AVC, ou environ 6 heures après le début de l'AVC, ou environ 8 heures après le début de l'AVC, ou
dans laquelle une thrombectomie est réalisée, facultativement dans laquelle la thrombectomie est réalisée environ 10 heures après début de l'AVC ou environ 12 heures après le début de l'AVC, ou jusqu'à 24 heures après le début de l'AVC,
et/ou dans laquelle le patient reçoit de 1 à 5 doses de la composition dans les 24 heures, facultativement avec au moins une dose avant la thérapie thrombolytique ou la thrombectomie, et au moins une dose après la thérapie thrombolytique ou la thrombectomie,
et/ou dans laquelle la composition est administrée par voie intraveineuse ou intra-artérielle, facultativement dans laquelle la composition est administrée par voie intra-artérielle sélectivement au cerveau précédemment hypoperfusé, ou dans laquelle la composition est administrée par voie intrathécale, ou dans laquelle la composition est administrée par tube intragastrique ou intraduodénal.

14. Composition pour utilisation selon la revendication 10, dans laquelle le patient souffre de ou présente un risque de lésion cérébrale traumatique, facultativement dans laquelle le patient se voit administrer la composition dans un délai d'environ 1 heure après la lésion cérébrale, ou dans un délai d'environ 2 heures après la lésion cérébrale, ou dans un délai d'environ 12 heures après la lésion cérébrale, ou dans un délai d'environ 24 heures après la lésion cérébrale, facultativement dans laquelle le patient se voit administrer la composition 1 à 10 fois, avec des fréquences comprises entre environ une fois toutes les quatre heures et environ une fois par semaine,
ou dans laquelle le patient souffre d'un trouble de stress post-traumatique (TSPT), facultativement dans laquelle le patient se voit administrer la composition environ une fois par semaine,
ou dans laquelle le patient souffre d'une lésion de la moëlle épinière, facultativement dans laquelle le patient se voit administrer la composition dans un délai d'environ 1 heure ou d'environ 2 heures après la lésion, et/ou dans laquelle le patient se voit administrer la composition au moins une fois par semaine pendant au moins quatre semaines,
ou dans laquelle le patient souffre d'une lésion aiguë d'un organe,
ou dans laquelle le patient souffre d'une maladie neurodégénérative, facultativement dans laquelle la maladie neurodégénérative est une SLA, une sclérose en plaques, une maladie de Parkinson, une maladie d'Alzheimer, ou une maladie de Huntington, et/ou dans laquelle le patient se voit administrer la composition au moins une fois par semaine et/ou se voit administrer pendant des périodes de récidive.

15. Composition pour utilisation selon la revendication 10, dans laquelle le patient reçoit en outre un traitement de supplémentation par voie orale avec des AG n-3, facultativement sous forme de DG ou TG.
